(19) Europäisches Patentamt European Patent Office Office européen des brevets

(11) **EP 2 854 668 B1**

(12) **FASCICULE DE BREVET EUROPEEN**

(45) Date de publication et mention de la délivrance du brevet:
**24.08.2016 Bulletin 2016/34**

(21) Numéro de dépôt: **13726782.9**

(22) Date de dépôt: **03.06.2013**

(51) Int Cl.:
**A61B 90/00** (2016.01)

(86) Numéro de dépôt international:
**PCT/EP2013/061382**

(87) Numéro de publication internationale:
**WO 2013/178823 (05.12.2013 Gazette 2013/49)**

(54) **DISPOSITIF DE GUIDAGE D'UNE SONDE D'IMAGERIE MEDICALE ET PROCEDE DE GUIDAGE D'UNE TELLE SONDE**

VORRICHTUNG ZUM FÜHREN EINER MEDIZINISCHEN BILDGEBUNGSSONDE UND VERFAHREN ZUM FÜHREN EINER SOLCHEN SONDE

DEVICE FOR GUIDING A MEDICAL IMAGING PROBE AND METHOD FOR GUIDING SUCH A PROBE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.06.2012 FR 1255130**

(43) Date de publication de la demande:
**08.04.2015 Bulletin 2015/15**

(73) Titulaire: **Koelis**
**38240 Meylan (FR)**

(72) Inventeurs:
• **BAUMANN, Michael**
**F-38100 Grenoble (FR)**
• **LEROY, Antoine**
**F-38240 Meylan (FR)**

(74) Mandataire: **Decorchemont, Audrey Véronique Christèle et al**
**CABINET BOETTCHER**
**16, rue Médéric**
**75017 Paris (FR)**

(56) Documents cités:
**WO-A1-2011/094585      WO-A2-2011/161684**
**FR-A1- 2 920 961      JP-A- 2011 104 108**
**US-A- 6 045 508      US-A1- 2008 262 356**
**US-A1- 2009 306 509      US-A1- 2011 184 684**
**US-B1- 6 248 074**

EP 2 854 668 B1

**Description**

L'invention concerne un dispositif de guidage d'une sonde d'imagerie médicale et un procédé de guidage d'une telle sonde.

ARRIERE-PLAN DE L'INVENTION

Les opérations dites « ouvertes » peuvent s'avérer lourdes pour un patient. Ainsi, de plus en plus, les praticiens ont recours à des opérations dites « mini-invasives » lors desquelles des instruments médicaux sont insérés à travers la peau (percutané), ou dans une voie naturelle du patient (vagin, rectum, conduit auditif...) ou dans une voie artificielle rapportée sur le corps du patient (canule, veine artificielle, trocart...). Pour cela, les praticiens s'aident d'images de l'organe où des organes concernés, les images étant prises avant ou pendant l'intervention.

En urologie, afin de dépister un éventuel cancer de la prostate, il est connu de réaliser une biopsie prostatique. Il s'agit de prélever des échantillons de tissus au sein même de la prostate, lesdits échantillons étant ensuite analysés en laboratoire afin de détecter la présence d'éventuelles cellules cancéreuses. A cet effet, le patient est couché sur le flanc ou sur le dos. Dans le cas d'une biopsie transrectale, un instrument médical comportant un porte-aiguille portant une aiguille à biopsie est inséré dans la voie naturelle qu'est le rectum. Grâce à l'instrument médical, le clinicien perce la paroi du colon pour atteindre la prostate et prélever ainsi des échantillons de tissus prostatiques. Pour le prélèvement, l'échographie bidimensionnelle de la prostate, prise pendant l'intervention sous forme de flux d'images, est typiquement utilisée par le clinicien pour positionner l'aiguille par rapport à la prostate. Or l'utilisation d'une image bidimensionnelle pour effectuer un positionnement tridimensionnel, le caractère relativement peu discriminant de l'échographie ainsi que la forme plutôt symétrique de la prostate et sa mobilité entraînent souvent une erreur significative de positionnement de l'aiguille par rapport à la prostate de sorte que les échantillons ne peuvent véritablement être prélevés ni de manière régulière ni de manière ciblée, selon les approches.

Pour aider le clinicien à prélever les échantillons de tissus aux lieux propices, il existe de nombreux dispositifs de guidage de l'instrument médical.

Par exemple, il est connu des dispositifs de guidage comportant un bras articulé commandé pour déplacer l'extrémité proximale de l'instrument. La position de l'extrémité proximale dans le repère général de la base fixe du bras articulé est donc toujours déterminable. En connaissant la position de la prostate dans le repère général, il est ainsi possible de connaître la position de l'instrument relativement à la prostate tout le long de l'intervention.

Toutefois, un tel dispositif de guidage est particulièrement encombrant.

En outre, un tel dispositif de guidage ne prend en compte que les mouvements de l'instrument. Or le patient peut également bouger lors de l'intervention ce qui entraîne un déplacement de la prostate. Par exemple, il est possible que le patient ne soit pas sous anesthésie locale et/ou qu'il ne soit pas immobilisé de sorte qu'il puisse bouger pour trouver une position plus confortable ou simplement par réflexe. La simple activité musculaire du patient comme son cycle respiratoire peut également entraîner un déplacement de la prostate. De plus, la prostate est un organe mou de sorte que la pression de l'instrument, ou même le simple contact de l'aiguille, sans que celle-ci soit enfoncée dans la prostate, suffit à déplacer ladite prostate. En outre, la vessie se remplit et grossit au cours de l'intervention. Elle peut alors venir appuyer sur la prostate de sorte à déplacer celle-ci. Des hématomes ou des accumulations de liquide, causés ou non par l'intervention, peuvent également apparaître et provoquer un déplacement de la prostate.

Ainsi, même si la position de l'instrument dans le repère général est correctement déterminée, la prostate peut bouger de sorte que le clinicien peut ne pas atteindre avec l'aiguille la région initialement visée.

Il est également connu des dispositifs de guidage comprenant un ou plusieurs émetteurs (ou marqueurs) solidaires de l'instrument et un récepteur (ou détecteur des marqueurs) qui est disposé dans la pièce où se situe le patient. Par exemple, les émetteurs génèrent des courants induits que le récepteur peut détecter ce qui permet de positionner les émetteurs, et donc l'instrument, dans le repère général du récepteur. En connaissant la position de la prostate dans le repère général du récepteur, il est ainsi possible de connaître la position de l'instrument relativement à la prostate au cours de l'intervention.

Or, de la même façon, un tel dispositif de guidage est encombrant et ne prend en compte que les mouvements de l'instrument et aucunement les éventuels déplacements de la prostate : la position relative de la prostate par rapport à l'instrument est en réalité rapidement décalée donc perdue. Dans d'autres cas il est possible d'ajouter des capteurs supplémentaires sur un volume anatomique ciblé par une intervention afin de résoudre ce problème, mais cette solution invasive n'est pas appuicable à une intervention sur la prostate qui est un organe mou et interne.

Récemment, une nouvelle catégorie de dispositifs de guidage est apparue permettant au clinicien de réaliser plus précisément les ponctions dans la prostate. En effet, lesdits dispositifs de guidage comportent une sonde échographique pourvue de moyens d'acquisition d'images en trois dimensions. Ladite sonde est agencée dans le dispositif de guidage de sorte qu'une position de la sonde relativement à l'instrument soit connue ou du moins toujours déterminable. Par exemple, la sonde est solidaire de l'instrument.

La sonde fournit alors en continu des images de la voie naturelle, des tissus environnants et de la prostate lors du déplacement de l'instrument dans la voie naturelle. Ces images sont ensuite traitées afin de déterminer la position de la sonde, et donc de l'instrument, par rapport à la prostate. Comme les images contiennent des informations sur les déplacements de la prostate, le guidage de l'instrument est plus précis. La demande de brevet FR 2 920 961 décrit un tel dispositif de guidage.

Toutefois, les sondes échographique acquérant des images en trois dimensions présentent l'inconvénient de nécessiter plusieurs secondes pour l'acquisition d'une image. En outre, les images tridimensionnelles comportant une quantité importante d'informations, le traitement desdites images requiert en conséquence également quelques secondes. Il n'est donc pas possible de connaître la position de la sonde relativement à la prostate suffisamment rapidement après le début de l'acquisition d'une nouvelle image de sorte que le positionnement de l'instrument relativement à la prostate reste encore trop imprécis. En outre, cette approche augmente la durée de l'intervention.

Afin de pallier les inconvénients précités, une troisième catégorie de dispositifs de guidage combinant la localisation de l'instrument par les images prises par une sonde échographique (deux dimensions, trois dimensions...) et la localisation par un ensemble émetteur/récepteur sont connus.

L'ensemble émetteur/récepteur permet de fournir rapidement une position approximative de la sonde, et donc de l'instrument, relativement à la prostate. Cette position est utilisée pour initialiser le traitement des images fournies par la sonde, le traitement des images permettant alors d'affiner la position de la sonde relativement à la prostate.

Ainsi, la position de la sonde, et donc de l'instrument, relativement à la prostate est régulée autour d'une position globale déterminée rapidement par le système émetteur/récepteur.

Un tel dispositif de guidage permet de localiser très précisément la sonde, et donc l'instrument, relativement à la prostate mais nécessite à la fois un système sonde/traitement d'images et un système émetteur/récepteur. De plus, le récepteur est souvent encombrant. En outre, des perturbations externes, comme des interférences magnétiques, peuvent gêner la localisation des récepteurs.

Il a alors été envisagé de remplacer le système émetteur/récepteur par un capteur porté par la sonde échographique.

Toutefois, de tels dispositifs s'avèrent peu fiables notamment en cas de mouvements importants de la prostate entre la capture d'une image de référence et d'une image de suivi ultérieure. En effet, avec de tels dispositifs, le traitement des images ne permet d'affiner la position fournie par le capteur qu'avec des méthodes d'optimisation locale. Or si l'organe se déplace de façon importante parce que le patient bouge pendant l'intervention, la position fournie par le capteur peut se trouver en dehors de ce qui est appelé « bassin d'attraction » (« capture range » en anglais) de la solution recherchée (soit la position réelle de la sonde relativement à la prostate) et il devient alors impossible pour un calcul par optimisation locale d'atteindre cette solution. Dans ce cas, ces dispositifs sont alors aussi peu performants que les dispositifs de guidage ne comprenant qu'un capteur seul puisqu'ils peuvent fournir des positions grossièrement erronées et plausiblement dangereuses. En outre, il s'avère nécessaire de calibrer régulièrement le dispositif de guidage pour le replacer selon une position connue relativement à la prostate ce qui est coûteux en temps et fastidieux pour l'opérateur.

Le document US 6 045 508 décrit une sonde d'imagerie médicale de l'art antérieur.

OBJET DE L'INVENTION

Un but de l'invention est d'obvier au moins en partie aux inconvénients précités.

BREVE DESCRIPTION DE L'INVENTION

A cet effet, on propose un dispositif de guidage d'une sonde d'imagerie médicale pour amener ladite sonde à proximité d'un volume anatomique, la sonde comportant des moyens d'acquisition d'images du volume anatomique, le dispositif comportant au moins un capteur monté solidaire de la sonde et apte à générer des signaux représentatifs d'au moins une rotation de la sonde dans l'espace. Selon l'invention, le dispositif comporte une unité de commande qui est reliée à la sonde et qui comporte des moyens de traitement des images pour situer la sonde relativement au volume anatomique, les moyens de traitement étant aptes à déduire une rotation de la sonde dans l'espace à partir des signaux générés par le capteur et comprenant des moyens d'estimation de plusieurs positions plausibles de la sonde relativement au volume anatomique à partir d'au moins la déduction de la rotation de la sonde dans l'espace, les moyens de traitement étant agencés pour déterminer la position de la sonde relativement au volume anatomique à partir d'une image de référence du volume anatomique, d'au moins une image acquise par les moyens d'acquisition d'images et les positions plausibles.

Ainsi, le capteur fournit des signaux permettant de déduire rapidement au moins une rotation de la sonde dans l'espace. Cette rotation est utilisée pour initialiser le calcul de la détermination d'une ou plusieurs positions plausibles de la sonde relativement au volume anatomique considéré. Ces positions plausibles sont alors elles-mêmes utilisées pour initialiser le traitement des images fournies par les moyens d'acquisition qui permet de déterminer la position de la sonde relativement au volume anatomique. L'estimation de la rotation dans l'espace permet donc au final d'initialiser par l'intermédiaire du calcul des positions plausibles le traitement des images fournies par les moyens d'acquisition.

En fournissant cette rotation de la sonde dans l'espace, le traitement des images est rendu plus robuste puisqu'il n'est plus nécessaire de déterminer la rotation de la sonde dans l'espace à partir des images. Par conséquent, le traitement des images requiert moins d'informations provenant des moyens d'acquisition pour déterminer la position complète de la sonde relativement au volume anatomique. Il devient donc possible de travailler avec un plus faible nombre d'informations provenant des moyens d'acquisition d'images ce qui permet d'accélérer le temps consacré à l'acquisition des images mais également d'accélérer le traitement en lui-même des images. Grâce à l'invention, on connaît ainsi précisément et rapidement la position de la sonde relativement au volume anatomique tout en s'affranchissant d'un système encombrant du type émetteur/récepteur de l'art antérieur, et ce malgré les possibles déplacements du volume anatomique.

Un autre avantage de l'invention est donc qu'une quantité plus faible d'informations provenant des moyens d'acquisition est nécessaire comparé à des dispositifs de guidage de l'art antérieur se basant uniquement sur le traitement des images pour estimer la position de la sonde relativement au volume anatomique considéré. Ceci permet d'accélérer le processus d'acquisition d'images par les moyens d'acquisition d'images et en conséquence de connaître la position de la sonde relativement au volume anatomique suffisamment rapidement après le début de l'acquisition d'une nouvelle image de sorte que le positionnement de la sonde relativement à la prostate soit précis.

Ainsi, l'association d'un capteur générant des signaux représentatifs d'une position partielle de la sonde, de moyens de traitement d'images et de moyens d'estimation permet à la fois d'avoir un suivi rapide de la position de la sonde relativement au volume anatomique grâce au capteur et à la fois d'avoir un suivi fiable de ladite position même lors de bougés considérables du volume anatomique ciblé grâce aux moyens d'estimation d'images.

Estimer des positions plausibles de la sonde relativement au volume anatomique à partir d'une information de la position de la sonde dans l'espace fournie par le capteur permet de limiter le traitement des images et d'éviter des situations dans lesquelles, le traitement des images ne permet pas d'obtenir la position réelle de la sonde relativement au volume anatomique comme avec certains dispositifs de l'art antérieur.

Selon un mode de réalisation préféré, le capteur est apte à générer uniquement des signaux représentatifs de la rotation de la sonde dans l'espace.

Ainsi, les moyens d'estimation déterminent les positions plausibles de la sonde relativement au volume anatomique uniquement à partir d'informations sur la rotation de la sonde dans l'espace soit sans information sur la translation de la sonde dans l'espace. Quand un patient est allongé lors de l'intervention, il est relativement probable qu'il se déplace de quelques centimètres sur son lit, mais il est bien moins probable que le volume anatomique tourne de plus de quelques degrés. Grâce à ce mode de réalisation, on n'utilise que des informations concernant la rotation de la sonde soit les informations qui ont le moins de probabilités d'être grandement modifiées au cours de l'intervention. Les moyens d'estimation permettent alors d'estimer les positions plausibles de la sonde vis-à-vis du volume anatomique et donc d'estimer les translations de la sonde dans l'espace ayant une forte probabilité de correspondre à la position réelle de la sonde relativement au volume anatomique.

Par volume anatomique on entend ici aussi bien un organe comme le rein, le sein, l'utérus, la thyroïde, le foie ou la prostate qu'une cavité comme par exemple le cul de sac de Douglas.

L'invention concerne également un procédé de guidage d'une sonde médicale d'imagerie médicale pour amener la sonde à proximité d'un volume anatomique, la sonde comportant des moyens d'acquisition d'images du volume anatomique et le procédé comportant l'étape de monter solidairement à la sonde au moins un capteur apte à générer des signaux représentatifs d'au moins une rotation de la sonde dans l'espace. Selon l'invention le procédé comporte les étapes de :

- traiter les signaux générés par le capteur pour en déduire au moins une rotation de la sonde dans l'espace,
- estimer plusieurs positions plausibles de la sonde relativement au volume anatomique à partir d'au moins la déduction de la rotation de la sonde dans l'espace,
- traiter les images pour situer la sonde relativement au volume anatomique en déterminant la position de la sonde relativement au volume anatomique à partir d'une image de référence du volume anatomique, d'au moins une image acquise par les moyens d'acquisition d'images et des positions plausibles déterminées.

BREVE DESCRIPTION DES DESSINS

L'invention sera mieux comprise à la lumière de la description qui suit d'un mode de réalisation particulier non limitatif de l'invention. Il sera fait référence aux figures ci-jointes parmi desquelles :

- la figure 1 est une vue schématique d'un dispositif de guidage selon l'invention inséré en partie dans le corps d'un patient ;

- la figure 2 est une vue agrandie en coupe d'une partie du dispositif de guidage illustré à la figure 1 ;
- la figure 3 est un schéma illustrant les différentes étapes d'un mode de mise en oeuvre particulier du procédé de guidage d'une sonde du dispositif illustré à la figure 1.

## DESCRIPTION DETAILLEE DE L'INVENTION

L'invention est illustrée en application à une biopsie de la prostate. Cette application n'est bien sûre pas limitative. En outre, l'invention est illustrée en application à un porte-aiguille. Cette application n'est pas non plus limitative.

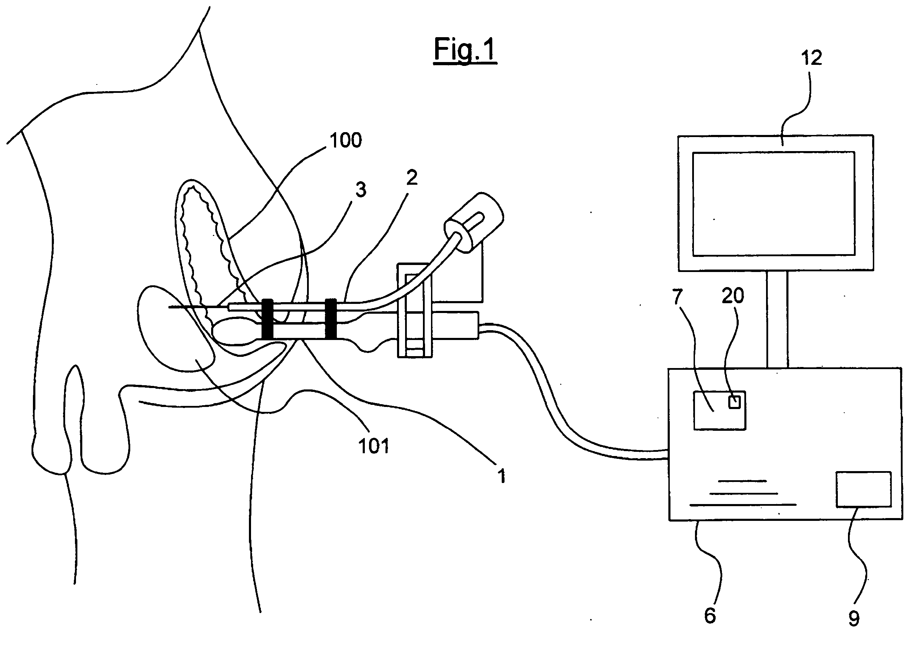

En référence à la figure 1, le dispositif de guidage selon l'invention comporte une sonde d'imagerie médicale 1 qui est illustrée insérée dans le rectum 100 d'un patient. Ici, le dispositif de guidage est destiné à guider la sonde 1 à proximité de la prostate 101 du patient. La sonde 1 comporte des moyens d'acquisition d'images de la prostate 101.

Selon un mode de réalisation particulier, la sonde 1 est rigidement liée à un instrument médical comportant ici un porte-aiguille 2 afin de réaliser une biopsie de la prostate 101. Le porte-aiguille 2 porte une aiguille 3. La sonde 1 étant solidaire du porte-aiguille 2, la position relative de la sonde 1 vis-à-vis du porte-aiguille 2 est connue.

Le dispositif de guidage comporte une unité de commande 6 à laquelle est reliée la sonde 1.

De préférence, le dispositif de l'invention comporte un écran de visualisation 12 des images acquises par la sonde 1. L'écran de visualisation 12 est relié à l'unité de commande 6. Ainsi, un praticien peut avoir un retour visuel des images prises de la prostate 101 ou d'une zone particulière de la prostate 101.

Selon un mode de réalisation privilégié, l'unité de commande 6 comporte des moyens de mémorisation 9 des images acquises par la sonde.

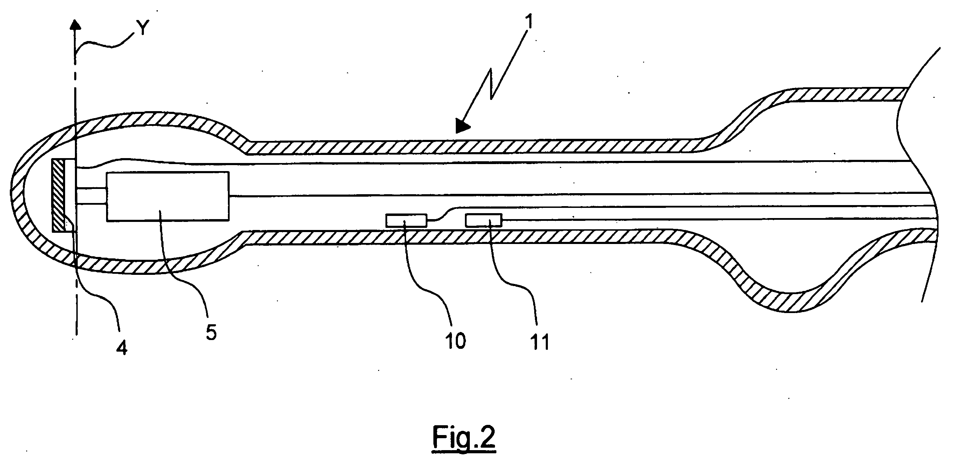

En référence à la figure 2, selon un mode de réalisation privilégié, les moyens d'acquisition comportent une barrette échographique 4 permettant d'acquérir des images en deux dimensions. Les moyens d'acquisition comportent en outre ici un moteur 5 qui est agencé dans la sonde 1 de sorte qu'une rotation de l'axe du moteur 5 entraîne une rotation de la barrette échographique 4 autour d'un premier axe Y.

De préférence, le moteur 5 est un moteur pas à pas ce qui permet de déplacer précisément, car sans accumulation d'erreur, la barrette échographique 4. Les images acquises par la barrette échographique 4 sont donc de meilleure qualité. En outre, le moteur 5 peut ainsi amener la barrette échographique 4 dans une position précise même sur une petite plage d'angle de rotation autour du premier axe Y.

Le moteur 5 et la barrette échographique 4 sont reliés, par exemple de façon filaire, à l'unité de commande 6.

Selon un mode de réalisation privilégié, l'unité de commande 6 contrôle le moteur 5 et la barrette échographique de sorte que le moteur 5 déplace la barrette échographique 4 dans une plage de rotation restreinte autour du premier axe Y, typiquement dans une plage de rotation de 20°, lorsque la barrette échographique 4 acquiert des images.

De façon privilégiée, l'unité de commande 6 contrôle le moteur 5 et la barrette échographique 4 de sorte que la barrette échographique 4 acquiert un faible nombre d'images, typiquement trois images, lors sa rotation par le moteur 5. De préférence, la barrette échographique 4 acquiert les images à des angles de rotation répartis régulièrement sur la plage de rotation autour du premier axe Y.

Selon un mode de réalisation privilégié, l'unité de commande 6 contrôle le moteur 5 et la barrette échographique 4 afin que le moteur 5 amène la barrette échographique dans une position initiale dite position de référence et bloque la barrette échographique 4 dans cette position de référence le temps pour la barrette échographique 4 d'acquérir au moins une image. Le moteur 5 entraîne alors en rotation la barrette échographique 4 sur une plage de rotation centrée autour de cette position de référence, la barrette échographique 4 acquérant d'autres images au cours de cette rotation. De façon encore plus privilégiée, le moteur 5 est commandé de sorte à bloquer la barrette échographique 4 dans chaque position prédéterminée où la barrette échographique 4 doit acquérir une image, le temps pour la barrette échographique d'acquérir ladite image.

Selon un mode de réalisation privilégié, l'unité de commande 6 contrôle le moteur 5 et la barrette échographique 4 de sorte que, après une première série d'acquisition d'images, le moteur 5 amène la barrette échographique 4 dans une position initiale pour une deuxième série d'acquisition d'images, ladite position initiale étant déterminée au moins à partir des images de la première série.

Ainsi, les images de la deuxième série sont acquises pour des positions bien particulières de la barrette échographique 4, afin de focaliser une collecte d'informations sur une zone de la prostate 101 à étudier plus en détail, zone identifiée à l'aide de la première série d'images. Ceci permet notamment de maximiser une qualité de localisation de la sonde 1 relativement à ladite zone.

Selon l'invention l'unité de commande 6 comporte des moyens de traitement 7 des images acquises par les moyens d'acquisition d'images.

De plus, le dispositif comporte au moins un premier capteur 10 solidaire de la sonde 1 et apte à générer des signaux exploitables par les moyens de traitement 7 afin d'en déduire au moins une rotation de la sonde 1 dans l'espace. Le premier capteur 10 est par exemple relié de façon filaire à l'unité de commande 6. De préférence, le premier capteur 10 comporte au moins un gyroscope générant des signaux exploitables qui permettent de calculer une position angulaire de la sonde 1 dans l'espace.

Ainsi, à partir des signaux générés par le premier capteur 10, la rotation de la sonde 1 dans l'espace est déterminée en temps réel. Ainsi, la détermination de la position de la sonde 1 relativement à la prostate 101 et donc ici du porte-aiguille 2 relativement à la prostate 101, est accélérée. La prostate 101 n'ayant que peu de mouvements propres de rotation, la simple connaissance de la rotation de la sonde 1 dans l'espace permet d'estimer de façon déjà précise la position de la sonde 1 relativement à la prostate 101.

En outre, les moyens de traitement 7 comportent des moyens d'estimation 20 de positions plausibles de la sonde 1 permettant de calculer un ensemble de positions plausibles de la sonde par rapport à la prostate à partir de la position angulaire de la sonde 1 dans l'espace c'est à dire à partir d'une position partiellement connue de la sonde dans l'espace.

Dès lors, à partir d'une image de référence et du traitement des images acquises par les moyens d'acquisition et des positions plausibles de la sonde 1 dans l'espace, les moyens de traitement 7 déterminent en service la position de la sonde 1 relativement à la prostate 101. La position relative de la sonde 1 vis-à-vis du porte-aiguille 2 étant connue, les moyens de traitement 7 permettent ici également de déterminer la position du porte-aiguille 2 relativement à la prostate 101.

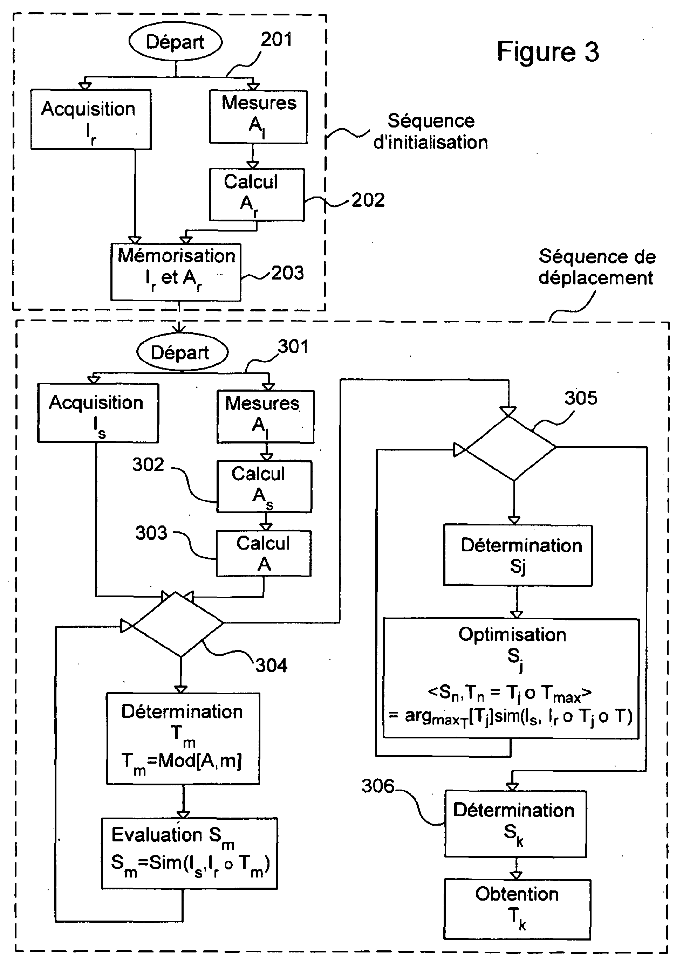

En référence à la figure 3, selon un mode de réalisation particulier, la position de la sonde 1 relativement à la prostate 101 est déterminée comme suit.

Lors d'une séquence d'initialisation, au cours d'une première étape 201, les moyens d'acquisition acquièrent une première image dite image de référence $I_r$ et la transmettent aux moyens de traitement 7. L'image de référence est par exemple une image générale de la prostate 101 ou bien une image d'une zone particulière de la prostate 101. Simultanément, le premier capteur 10 génère des signaux exploitables pour déterminer une position angulaire en temps réel de la sonde 1. De préférence, les moyens de traitement 7 déterminent en temps réel les L positions angulaires $A_1$ de la sonde 1 durant le temps qu'a duré l'acquisition de l'image de référencé $I_r$. Dans une deuxième étape 202, à partir des L positions angulaires $A_1$ déterminées à la première étape 201, les moyens de traitement 7 calculent la position angulaire moyenne $A_r$ de la sonde 1 par la formule suivante :

$$A_r = (1/L)\sum A_l$$

Dans une troisième étape 203, les moyens de traitement 7 mémorisent l'image de référence $I_r$ ainsi que la position angulaire moyenne $A_r$ de la sonde 1 associée à cette image de référence $I_r$.

Lors d'une séquence de déplacement de la sonde 1, au cours d'une première étape 301, les moyens d'acquisition acquièrent au moins une image $I_s$ et la transmettent aux moyens de traitement 7. Simultanément, le premier capteur 10 génère des signaux exploitables pour déterminer une position angulaire en temps réel de la sonde 1. De préférence, les moyens de traitement 7 déterminent en temps réel les L positions angulaires $A_1$ de la sonde 1 durant le temps qu'a duré l'acquisition de l'image $I_s$. Dans une deuxième étape 302, à partir des L positions angulaires $A_1$ déterminées à la première étape 301, les moyens de traitement 7 calculent la position angulaire moyenne $A_s$ de la sonde 1 par la formule suivante :

$$A_s = (1/L)\sum A_l$$

Lors d'une troisième étape 303, les moyens de traitement 7 déterminent la position angulaire relative A de la sonde par la formule :

$$A = (A_s)^{-1} A_r$$

On rappelle que $A_r$ est la position angulaire moyenne de référence de la sonde 1 et $A_s$ la position angulaire moyenne de la sonde 1 associée à la séquence de déplacement de la sonde 1.

Lors d'une quatrième étape 304, les moyens d'estimation 20 mettent en oeuvre l'algorithme suivant.

Pour chaque entier m compris entre 1 et M, M étant un entier prédéterminé par exemple par une analyse statistique de données de plusieurs tests initiaux, les moyens d'estimation 20 déterminent $T_m$ une position plausible de la sonde 1 relativement à la prostate 101 par la formule suivante :

$$T_m = Mod(A, m)$$

où Mod est un modèle statistique ou cinématique décrivant un nombre restreint de positions plausibles de la sonde 1 par rapport à la prostate 101 à partir de l'image de référence Ir.

Par exemple, Mod(A,m) peut définir un échantillonnage régulier de translation pour une orientation donnée par la formule suivants :

$$Mod(A, m) = \begin{cases} t_x = (m \ mo\,dulo \ x) * S_x - x_0 \\ t_y = ((m/x) \ mo\,dulo \ y) * S_y - y_0 \\ t_z = m/(x*y) * S_z - z_0 \end{cases}$$

de sorte que la position $T_m$ soit alors définie par

$$T_m(x, y, z) = A(x, y, z) + (t_x \quad t_y \quad t_z)^T$$

$x_0$, $y_0$, $z_0$ définissant le centre de la zone d'échantillonnage,
$S_x$, $S_y$, $S_z$ définissant l'échelonnage de la zone d'échantillonnage.

Les paramètres x, y, z ; $S_x$, $S_y$, $S_z$ et $x_0$, $y_0$, $z_0$ sont par exemple déterminés par une analyse statistique.
Puis, les moyens de traitement 7 évaluent un paramètre de similarité $S_m$ entre l'image $I_s$ acquise en début de séquence de déplacement et l'image de référence $I_r$ transformée à partir de la position $T_m$ selon la formule:

$$S_m = Sim(I_s, I_r \circ T_m)$$

où, Sim est une fonction de mesure de la distance entre deux images ou deux sous-ensembles d'images. Sim est par exemple une fonction de mesure de la corrélation croisée de deux images (ou de deux sous-ensembles d'images) qui est déterminée sur les niveaux de gris des images (ou des éléments des deux sous-ensembles d'images).
Ainsi, lors de la quatrième étape 304, à partir des informations de la rotation de la sonde 1 dans l'espace et d'un modèle statistique ou cinématique Mod, les moyens d'estimation 20 prédisent un nombre limité mais suffisamment exhaustif de positions plausibles de la sonde 1 relativement à la prostate 101. Les moyens de traitement 7 estiment également pour chaque position plausible, un paramètre de similarité entre l'image acquise en début de séquence de déplacement et une transformation de l'image de référence à partir de la position plausible associée. A chaque position plausible $T_m$ est ainsi associé un paramètre de similarité $S_m$.
Lors d'une cinquième étape 305, les moyens de traitement 7 mettent en oeuvre l'algorithme suivant.
Pour chaque entier n compris entre 1 et N, N étant un entier prédéterminé par une analyse statistique de données de plusieurs tests initiaux, les moyens de traitement 7 déterminent le paramètre de similarité $S_j$ correspondant au n-ième meilleur résultat dans la liste des paramètres de similarités $\{S_1, ..., S_m\}$ calculés au cours de la quatrième étape 304.
Puis les moyens de traitement 7 effectuent une optimisation locale sur le paramètre de similarité $S_j$ rietenu en faisant varier la position $T_j$ associée, jusqu'à ce qu'un paramètre de similarité maximale $S_n$ soit trouvé pour une position $T_n$ qui est située dans l'environnement de $T_j$. Les moyens de traitement utilisent par exemple la formule suivante :

$$\langle S_n, T_n = T_j \circ T_{max} \rangle = \arg\max{}_T [T_j] Sim(I_s, I_r \circ T_j \circ T)$$

où T est la variable d'optimisation (on fait varier la position T pour trouver autour de $T_j$ une position $T_{max}$ qui maximise la similarité).
Des techniques d'optimisation locales comme la descente de gradient conjugué ou encore Powell-Brent sont bien connues de l'art antérieur et pourront être employées pour l'optimisation locale sur le paramètre de similarité $S_j$ précité.

Ainsi, lors de la cinquième étape 305, les moyens de traitement 7 testent et classifient les positions $T_m$ plausibles de la sonde 1 relativement à la prostate 101 calculées au cours de la quatrième étape 304. Les positions $T_m$ associées à un paramètre de similarité maximal sont retenues en tant que candidats ayant une forte probabilité d'être proches de la position réelle de la sonde 1 par rapport à la prostate 101. Ces positions à paramètre de similarité maximal sont ensuite étudiées plus en détail en effectuant une optimisation locale sur le paramètre de similarité pour chaque position à paramètre de similarité maximal retenu.

On sélectionne ainsi uniquement certaines positions $T_m$ fournies par les moyens d'estimation afin de réduire le nombre de minimisations effectuées lors de l'optimisation locale.

Puis, lors d'une sixième étape 306, les moyens de traitement 7 déterminent le paramètre de similarité maximal $S_k$ parmi tous les paramètres de similarité qui ont été déterminés lors de la cinquième étape 305. La position $T_k$ associée à ce paramètre de similarité maximal $S_k$ est la position déterminée de la sonde 1 par rapport à la prostate 101. Cette position $T_k$ est utilisée par exemple pour guider la sonde 1 relativement à la prostate 101.

Le traitement d'image consiste ici en la combinaison d'un calcul de similarité entre images avec une optimisation locale (basé par exemple sur les méthodes Powell-Brent ou de descente de gradient). L'étape d'optimisation consiste à partir d'une ou plusieurs positions initiales et la/les fait varier jusqu'à ce que la similarité entre les deux images à superposer anatomiquement soit localement maximale.

En référence à la figure 2, selon un mode de réalisation privilégié, le dispositif comporte un deuxième capteur 11, qui est solidaire de la sonde 1, apte à générer des signaux exploitables par les moyens de traitement 7 pour en déduire également une translation de la sonde 1 dans l'espace.

A partir des signaux générés par le premier capteur 10 et le deuxième capteur 11, la rotation et la translation de la sonde 1 dans l'espace sont déterminés en temps réel ce qui accélère encore davantage la détermination de la position de la sonde 1, et donc du porte-aiguille 2, relativement à la prostate 101 une fois une image, acquise.

Le premier capteur 10 et le deuxième capteur 11 sont peu encombrants et permettent de simplifier considérablement des calculs des moyens de traitement 7.

Le dispositif selon l'invention permet de connaître précisément et rapidement la position de la sonde relativement au volume anatomique tout en s'affranchissant d'un système encombrant du type émetteur/récepteur de l'art antérieur, et ce malgré les possibles déplacements du volume anatomique. Le dispositif permet ainsi de guider précisément et rapidement la sonde relativement au volume anatomique mais également ici de guider précisément et rapidement l'instrument relativement au volume anatomique. De façon avantageuse, le ou les capteurs solidaires de la sonde sont légers, de petites dimensions et agencés dans ou sur la sonde.

Le dispositif est en outre employable pour de nombreuses applications. Par exemple, un praticien peut déplacer seul la sonde et/ou l'instrument en s'aidant des images acquises par la sonde et de la position relative de la sonde et de l'instrument vis-à-vis du volume anatomique pour guider la sonde et/ou l'instrument. En variante, le dispositif pourra comporter des moyens de déplacement de la sonde et/ou de l'instrument, les moyens de déplacement comportant par exemple un bras articulé. L'unité de commande générera des ordres de commande à destination des moyens de déplacement afin de contrôler au moins un déplacement de la sonde et/ou de l'instrument relativement au volume anatomique à partir des images acquises par la sonde et de la position de la sonde relativement au volume anatomique. En variante, les moyens de déplacement seront co-manipulables avec le praticien.

L'invention n'est pas limitée à ce qui vient d'être décrit, mais bien au contraire englobe toute variante entrant dans le cadre défini par les revendications.

Bien que l'invention soit illustrée en application à une biopsie d'une prostate, l'invention pourra être utilisée dans d'autres applications. Par exemple, l'invention pourra permettre le traitement d'une maladie de la prostate, un instrument associé à la sonde étant alors approché de la prostate par le périnée ou le rectum. L'invention pourra permettre une ponction d'un volume anatomique du système génital féminin, comme une ponction de l'utérus, un instrument associé à la sondé étant alors approché du volume anatomique par le vagin. L'invention pourra également permettre une ponction d'autre volume anatomique comme un rein, un rachis, un sein, un poumon ...

Si la sonde doit être insérée dans le corps du patient pour atteindre le volume anatomique voulu, la sonde pourra être introduite par une voie naturelle comme le rectum mais également par une voie artificielle. La sonde pourra également être guidée relativement au volume anatomique voulu sans être insérée dans le corps du patient selon l'application à laquelle on destine l'invention. En tout état de cause, l'invention est totalement indépendante du point d'entrée de la voie naturelle ou artificielle choisie.

La sonde pourra approcher un tout volume anatomique qu'une prostate comme un rein, un sein, ou le cul de sac de Douglas ...La sonde pourra ainsi être associée à un autre instrument qu'un porte-aiguille comme un scalpel, une pince, une sonde de chaleur ou une fibre lumineuse, .... La sonde pourra également n'être associée à aucun instrument, l'invention permettant alors l'étude d'une zone particulière d'un volume anatomique par la sonde. Dans le cas où le dispositif comporte un instrument médical, l'instrument médical pourra ne pas être rigidement lié à la sonde. Le dispositif comportera alors des moyens de localisation de l'instrument médical relativement à la sonde de sorte que les moyens de traitement déterminant la position de la sonde relativement au volume anatomique puissent également déterminer la position de l'instrument relativement au volume anatomique. Le dispositif selon l'invention permettra ainsi de guider l'instrument relativement au volume anatomique. Par exemple les moyens de localisation de l'instrument médical relativement à la sonde comporteront des moyens mécaniques de solidarisation de l'instrument médical à la sonde comme un bras articulé.

La sonde pourra comporter un autre nombre de capteurs et d'autres types de capteur. Ainsi, la sonde pourra ne comporter qu'un capteur permettant de déduire la rotation de la sonde dans l'espace, cette information étant la plus importante. Les mouvements de translations de la sonde dans l'espace devront alors être déterminés de façon traditionnelle par traitement des images acquises par les moyens d'acquisition. Le premier capteur pourra par exemple comporter au moins un inclinomètre et/ou au moins un accéléromètre et/ou au moins un gyromètre. Les capteurs pourront être solidaires de la sonde sans être agencés à l'intérieur de la sonde.

Les moyens d'acquisition d'images pourront être différents de ceux décrits. Par exemple, les moyens d'acquisition d'images pourront être de type optique et non échographique. Par exemple, les moyens d'acquisition pourront comporter deux barrettes échographiques agencées dans la sonde de sorte à se croiser orthogonalement mais ne comporteront pas de moteur. Le dispositif pourra ne pas comporter d'écran de visualisation des images prises par la sonde. De préférence, le dispositif comportera une interface de communication avec une personne utilisant ledit dispositif comme un écran de visualisation et/ou des moyens de communication auditif et/ou des moyens de retour haptique ...

Bien que l'on ait indiqué que l'unité de commande était agencée de sorte à contrôler le moteur et la barrette échographique afin que, en service, après une première série d'acquisition d'images, le moteur amène la barrette échographique dans une position initiale pour une deuxième série d'acquisition d'images, ladite position initiale étant déterminée au moins à partir des images de la première série, l'unité de commande pourra ne pas comporter une telle fonction. Pour certaines interventions, il est parfois nécessaire qu'une image illustrant au moins une partie du volume de référence avec au moins une partie d'un instrument médical associé à la sonde soit toujours acquise au cours d'une série d'acquisition d'images. Une telle image est en effet très utile au clinicien pour se repérer. Dans ces conditions, la position initiale pour la deuxième série d'acquisition d'images doit également être déterminée de sorte qu'une telle image soit acquise au cours de la deuxième série.

Le procédé de détermination de la position de la sonde pourra être différent de ce qui a été présenté. Seule une position angulaire de la sonde pourra être déterminée et non une position angulaire moyenne de la sonde. D'autres étapes de traitement des images pourront être ajoutées pour affiner la détermination de la position de la sonde 1 relativement à la prostate 101. Les entiers N et M pourront être déterminés lors de la séquence d'initialisation. Par exemple, les moyens de traitement 7 pourront poursuivre le traitement des images après l'étape de détermination de Tk par exemple en estimant des transformations résiduelles telles que les déformations causées par la sonde 1 à la prostate 101.

En variante, le procédé pourra comporter une étape supplémentaire de validation du résultat du calcul de la position de la sonde relativement au volume anatomique. Cette étape consistera par exemple à calculer une mesure de similarité complexe entre l'image de référence transformée par la position calculée de la sonde relativement au volume anatomique et l'image acquise par les moyens d'acquisition et de valider cette similarité par exemple par seuillage. Ceci permettra d'identifier un calcul erroné de la position de la sonde relativement au volume anatomique et éventuellement de prévenir l'utilisateur ou de lui proposer de nouvelles stratégies de localisation de la sonde relativement au volume anatomique.

Le modèle statistique ou cinématique mis en oeuvre par les moyens d'estimation pourra ainsi être différent de celui décrit. Le modèle statistique ou cinématique pourra ainsi être spécifique du type d'intervention visé voire même spécifique du type de catégorie à laquelle appartient le patient Le modèle pourra ainsi être un modèle cinématique des bougés plausibles de la sonde lors d'un accès transrectal à la prostate tel que décrit dans la demande de brevet FR 2 920 961 A1, un modèle statistique de type analyse des composants principaux (ACP) qui permet de décrire à partir d'une analyse statistique les principaux axes de variation et l'étendue des variations, un modèle construit selon les lois de Bayes ayant pour but d'identifier des positions de la sonde relativement au volume anatomique avec des probabilités a posteriori maximales en considérant les signaux générés par le capteur solidaire de la sonde. Le modèle considéré sera en général construits à partir d'un nombre statistiquement suffisant de cas cliniques représentatifs. En variante, les moyens d'estimation comporteront des moyens de calculs qui fourniront un sous-échantillonnage régulier des axes de l'espace de transformations rigides de la sonde dans l'espace, lesdits n'étant pas couverts par les signaux du capteur solidaire de la sonde et/ou ayant une forte probabilité d'être exposés au bougé du volume anatomique.

Les étapes de calcul de similarité peuvent être mises en oeuvre à partir d'un coefficient de corrélation (CC) ou la somme de distances absolues (SDA).

**Revendications**

1. Dispositif de guidage d'une sonde d'imagerie médicale (1) pour amener ladite sonde à proximité d'un volume anatomique (101), la sonde comportant des moyens d'acquisition d'images (4,5) du volume anatomique, le dispositif comportant au moins un capteur (10) monté solidaire de la sonde et apte à générer des signaux représentatifs d'au moins une rotation de la sonde dans l'espace, le dispositif étant **caractérisé en ce qu'**il comporte une unité de commande (6) qui est reliée à la sonde et qui comporte des moyens de traitement (7) des images pour situer la sonde relativement au volume anatomique, les moyens de traitement étant aptes à déduire une rotation de la sonde dans l'espace à partir des signaux générés par le capteur et comprenant des moyens d'estimation (20) de plusieurs positions plausibles de la sonde relativement au volume anatomique à partir d'au moins la déduction de la rotation de la sonde dans l'espace, les moyens de traitement étant agencés pour déterminer la position de la sonde relativement au volume anatomique à partir d'une image de référence du volume anatomique, d'au moins une image acquise par les moyens d'acquisition d'images et les positions plausibles.

2. Dispositif selon la revendication 1, dans lequel le capteur (10) est apte à générer uniquement des signaux représentatifs de la rotation de la sonde dans l'espace.

3. Dispositif selon la revendication 1, dans lequel les moyens de traitement (7) sont agencés pour déterminer uniquement une position angulaire de la sonde relativement au volume anatomique à partir des signaux générés par le capteur et les moyens d'estimation (20) sont agencés pour déterminer plusieurs positions plausibles de la sonde à partir de ladite position angulaire.

4. Dispositif selon la revendication 1, dans lequel les moyens d'acquisition d'images comportent une barrette échographique (4) et un moteur (5) agencé de sorte à, en service, entraîner en rotation la barrette échographique autour d'un axe de rotation (Y).

5. Dispositif selon la revendication 4, dans lequel l'unité de commande (6) est agencée de sorte à contrôler le moteur (5) et la barrette échographique (4) afin que, en service, le moteur déplace la barrette échographique dans une plage de rotation restreinte, typiquement de 20 degrés, autour de l'axe de rotation (Y).

6. Dispositif selon la revendication 4, dans lequel l'unité de commande (6) est agencée de sorte à contrôler le moteur (5) et la barrette échographique (4) afin que, en service, après une première série d'acquisition d'images, le moteur amène la barrette échographique dans une position initiale pour une deuxième série d'acquisition d'images, ladite position initiale étant déterminée au moins à partir des images de la première série.

7. Procédé de guidage d'une sonde médicale d'imagerie médicale (1) pour amener la sonde à proximité d'un volume anatomique (101), la sonde comportant des moyens d'acquisition d'images (4,5) du volume anatomique et le procédé comportant l'étape de monter solidairement à la sonde au moins un capteur (10) apte à générer des signaux représentatifs d'au moins une rotation de la sonde dans l'espace, le procédé étant **caractérisé en ce qu'**il comporte les étapes de :

   - traiter les signaux générés par le capteur pour en déduire au moins une rotation de la sonde dans l'espace,
   - estimer plusieurs positions plausibles de la sonde relativement au volume anatomique à partir d'au moins la déduction de la rotation de la sonde dans l'espace,
   - traiter les images pour situer la sonde relativement au volume anatomique en déterminant la position de la sonde relativement au volume anatomique à partir d'une image de référence du volume anatomique, d'au moins une image acquise par les moyens d'acquisition d'images et des positions plausibles déterminées.

8. Procédé selon la revendication 7, dans lequel l'étape d'estimer plusieurs position plausibles est réalisée par l'intermédiaire d'un modèle statistique ou cinématique (Mod(A,m)).

9. Procédé selon la revendication 7, dans lequel l'étape d'estimer plusieurs position plausibles est réalisée à partir de la déduction d'uniquement la rotation de la sonde dans l'espace

10. Procédé selon la revendication 7, comportant en outre l'étape de ne sélectionner que certaines positions plausibles de la sonde relativement au volume anatomique pour initialiser l'étape de traiter les images pour situer la sonde relativement au volume anatomique en déterminant la position de la sonde relativement au volume anatomique.

**11.** Procédé selon la revendication 7, comportant en outre l'étape de valider la position déterminée de la sonde relativement au volume anatomique.

**12.** Procédé selon la revendication 7, comportant les étapes successives de :

- acquérir au moins une image de référence du volume anatomique ($I_r$) et déterminer au moins une position angulaire de la sonde dans l'espace associée à l'image de référence dite position angulaire de référence ($A_r$); acquérir au moins une première image du volume anatomique ($I_s$) et déterminer au moins une première position angulaire ($A_s$) de la sonde dans l'espace associée à ladite image en traitant les signaux générés par le capteur pour en déduire la rotation de la sonde dans l'espace;
- déterminer à partir de l'image de référence ($I_r$), de la position angulaire de référence ($A_r$), de la première image ($I_s$) et de la première position angulaire ($A_s$), un nombre fini de positions ($T_m$) de la sonde relativement au volume anatomique par l'intermédiaire d'un modèle statistique ou cinématique ($Mod(A,m)$)de sorte à estimer plusieurs positions plausibles de la sonde relativement au volume anatomique;
- calculer pour chaque position plausible ($T_m$), un paramètre de similarité ($S_m$) entre l'image du volume anatomique ($I_s$) et une transformation de l'image de référence ($I_r$) à partir de ladite position plausible;
- à l'aide des paramètres de similarité ($S_m$) calculés à l'étape précédente, sélectionner parmi les positions plausibles ($T_m$) des positions ($T_j$) les plus proches d'une position réelle de la sonde relativement au volume anatomique ;
- affiner la détermination de chaque position ($T_n$) de la sonde relativement au volume anatomique par une optimisation locale sur chacun des paramètres de similarités associés aux positions ($T_j$);
- sélectionner, parmi toutes les positions ainsi affinées ($T_n$) la position ($T_k$) dont le paramètre de similarité associé ($S_k$) est maximal après l'étape d'optimisation locale, ladite position étant la position déterminée de la sonde relativement au volume anatomique.

**13.** Procédé selon la revendication 12, dans lequel le paramètre de similarité est calculé à partir d'une corrélation croisée sur des niveaux de gris entre l'image du volume anatomique ($I_s$) et la transformation de l'image de référence ($I_r$).

**Patentansprüche**

**1.** Vorrichtung zum Führen einer medizinischen Bildgebungssonde (1), um die genannte Sonde in die Nähe eines anatomischen Volumens (101) zu bringen, wobei die Sonde Bilderfassungsmittel (4, 5) zum Erfassen von Bildern des anatomischen Volumens umfasst, wobei die Vorrichtung mindestens einen Sensor (10) umfasst, der fest mit der Sonde verbunden und dazu geeignet ist, Signale zu erzeugen, die repräsentativ für mindestens eine Drehung der Sonde im Raum sind, wobei die Vorrichtung **dadurch gekennzeichnet ist, dass** sie eine Steuereinheit (6) umfasst, die mit der Sonde verbunden ist und die Bildverarbeitungsmittel (7) enthält, um die Sonde relativ zum anatomischen Volumen zu lokalisieren, wobei die Verarbeitungsmittel dazu geeignet sind, eine Drehung der Sonde im Raum anhand der von dem Sensor erzeugten Signale herzuleiten, und Schätzungsmittel (20) umfassen, um mehrere plausible Positionen der Sonde relativ zum anatomischen Volumen anhand mindestens der Herleitung der Drehung der Sonde im Raum zu schätzen, wobei die Verarbeitungsmittel so ausgebildet sind, dass sie die Position der Sonde relativ zum anatomischen Volumen anhand eines Referenzbildes des anatomischen Volumens, mindestens eines von den Bilderfassungsmitteln erfassten Bildes und den plausiblen Positionen bestimmen.

**2.** Vorrichtung nach Anspruch 1, wobei der Sensor (10) dazu geeignet ist, nur Signale zu erzeugen, die repräsentativ für die Drehung der Sonde im Raum sind.

**3.** Vorrichtung nach Anspruch 1, wobei die Verarbeitungsmittel (7) so ausgebildet sind, dass sie nur eine Winkelposition der Sonde relativ zum anatomischen Volumen anhand der von dem Sensor erzeugten Signale bestimmen, und die Schätzungsmittel (20) so ausgebildet sind, dass sie mehrere plausible Positionen der Sonde anhand der genannten Winkelposition bestimmen.

**4.** Vorrichtung nach Anspruch 1, wobei die Bilderfassungsmittel ein Ultraschall-Array (4) und einen Motor (5) umfassen, der so ausgebildet ist, dass er im Betrieb das Ultraschall-Array drehend um eine Drehachse (Y) antreibt.

**5.** Vorrichtung nach Anspruch 4, wobei die Steuereinheit (6) so ausgebildet ist, dass sie den Motor (5) und das Ultraschall-Array (4) steuert, damit der Motor im Betrieb das Ultraschall-Array in einem begrenzten Drehbereich, üblicherweise 20 Grad, um die Drehachse (Y) bewegt.

**6.** Vorrichtung nach Anspruch 4, wobei die Steuereinheit (6) so ausgebildet ist, dass sie den Motor (5) und das Ultraschall-Array (4) steuert, damit der Motor im Betrieb nach einer ersten Bilderfassungsreihe das Ultraschall-Array in eine Anfangsposition für eine zweite Bilderfassungsreihe bringt, wobei die genannte Anfangsposition mindestens anhand der Bilder der ersten Reihe bestimmt wird.

**7.** Verfahren zum Führen einer medizinischen Bildgebungssonde (1), um die Sonde in die Nähe eines anatomischen Volumens (101) zu bringen, wobei die Sonde Bilderfassungsmittel (4, 5) zum Erfassen von Bildern des anatomischen Volumens umfasst und das Verfahren den Schritt des festen Anbringens mindestens eines Sensors (10) an der Sonde umfasst, wobei der Sensor dazu geeignet ist, Signale zu erzeugen, die repräsentativ für mindestens eine Drehung der Sonde im Raum sind, wobei das Verfahren **dadurch gekennzeichnet ist, dass** es die Schritte umfasst:

- Verarbeiten der von dem Sensor erzeugten Signale, um daraus mindestens eine Drehung der Sonde im Raum herzuleiten,
- Schätzen mehrerer plausibler Positionen der Sonde relativ zum anatomischen Volumen anhand mindestens der Herleitung der Drehung der Sonde im Raum,
- Verarbeiten der Bilder, um die Sonde relativ zum anatomischen Volumen zu lokalisieren, indem die Position der Sonde relativ zum anatomischen Volumen anhand eines Referenzbildes des anatomischen Volumens, mindestens eines von den Bilderfassungsmitteln erfassten Bildes und der bestimmten plausiblen Positionen bestimmt wird.

**8.** Verfahren nach Anspruch 7, wobei der Schritt des Schätzens mehrerer plausibler Positionen mittels eines statistischen oder kinematischen Modells (Mod (A, m)) durchgeführt wird.

**9.** Verfahren nach Anspruch 7, wobei der Schritt des Schätzens mehrerer plausibler Positionen anhand der Herleitung nur der Drehung der Sonde im Raum durchgeführt wird.

**10.** Verfahren nach Anspruch 7, ferner umfassend den Schritt des Auswählens nur gewisser plausibler Positionen der Sonde relativ zum anatomischen Volumen, um den Schritt des Verarbeitens der Bilder zu initialisieren, um die Sonde relativ zum anatomischen Volumen zu lokalisieren, indem die Position der Sonde relativ zum anatomischen Volumen bestimmt wird.

**11.** Verfahren nach Anspruch 7, ferner umfassend den Schritt des Validierens der bestimmten Position der Sonde relativ zum anatomischen Volumen.

**12.** Verfahren nach Anspruch 7, umfassend die aufeinander folgenden Schritte:

- Erfassen mindestens eines Referenzbildes ($I_r$) des anatomischen Volumens und Bestimmen mindestens einer mit dem Referenzbild verbundenen Winkelposition der Sonde im Raum, die als Referenzwinkelposition ($A_r$) bezeichnet wird;
- Erfassen mindestens eines ersten Bildes des anatomischen Volumens ($I_s$) und Bestimmen mindestens einer mit dem genannten Bild verbundenen ersten Winkelposition ($A_s$) der Sonde im Raum, indem die von dem Sensor erzeugten Signale verarbeitet werden, um daraus die Drehung der Sonde im Raum herzuleiten;
- Bestimmen anhand des Referenzbildes ($I_r$), der Referenzwinkelposition ($A_r$), des ersten Bildes ($I_s$) und der ersten Winkelposition ($A_s$) einer endlichen Anzahl von Positionen ($T_m$) der Sonde relativ zum anatomischen Volumen mittels eines statistischen oder kinematischen Modells (Mod (A,m), derart, dass mehrere plausible Positionen der Sonde relativ zum anatomischen Volumen geschätzt werden;
- für jede plausible Position ($T_m$) Berechnen eines Ähnlichkeitsparameters ($S_m$) zwischen dem Bild des anatomischen Volumens ($I_s$) und einer Transformation des Referenzbildes ($I_r$) anhand der genannten plausiblen Position;
- mit Hilfe der Ähnlichkeitsparameter ($S_m$), die im vorhergehenden Schritt berechnet wurden, Auswählen unter den plausiblen Positionen ($T_m$) von Positionen ($T_j$), die einer tatsächlichen Position der Sonde relativ zum anatomischen Volumen am nächsten sind;
- Verfeinern der Bestimmung jeder Position ($T_n$) der Sonde relativ zum anatomischen Volumen durch eine lokale Optimierung über jeden der mit den Positionen ($T_j$) verbundenen Ähnlichkeitsparameter;
- Auswählen unter allen so verfeinerten Positionen ($T_n$) der Position ($T_k$), deren dazugehöriger Ähnlichkeitsparameter ($S_k$) nach dem Schritt der lokalen Optimierung maximal ist, wobei diese genannte Position die bestimmte Position der Sonde relativ zum anatomischen Volumen ist.

**13.** Verfahren nach Anspruch 12, wobei der Ähnlichkeitsparameter anhand einer Kreuzkorrelation an Graustufen zwischen dem Bild des anatomischen Volumens ($I_s$) und der Transformation des Referenzbildes ($I_r$) berechnet wird.

**Claims**

**1.** A device for guiding a medical imaging probe (1) in order to bring said probe in proximity to an anatomical volume (101), the probe comprising means (4, 5) for acquiring images of the anatomical volume, the device comprising at least one sensor (10) fixed to the probe and capable of generating signals representing at least one rotation of the probe in space, the device being **characterized in that** it comprises a control unit (6) which is connected to the probe and comprises means (7) for processing the images in order to localize the probe relative to the anatomical volume, the processing means being capable of deducing a rotation of the probe in space on the basis of the signals generated by the sensor, and comprising means (20) for estimating a plurality of plausible positions of the probe relative to the anatomical volume on the basis of at least the deduction of the rotation of the probe in space, the processing means being arranged in order to determine the position of the probe relative to the anatomical volume on the basis of a reference image of the anatomical volume, of at least one image acquired by the image acquisition means and the plausible positions.

**2.** The device as claimed in claim 1, wherein the sensor (10) is capable of generating only signals representing the rotation of the probe in space.

**3.** The device as claimed in claim 1, wherein the processing means (7) are arranged in order to determine only an angular position of the probe relative to the anatomical volume on the basis of the signals generated by the sensor, and the estimation means (20) are arranged in order to determine a plurality of plausible positions of the probe on the basis of said angular position.

**4.** The device as claimed in claim 1, wherein the image acquisition means comprise a linear ultrasound array (4), and a motor (5) which is arranged so as to drive the linear ultrasound array in rotation about a rotation axis (Y) during use.

**5.** The device as claimed in claim 4, wherein the control unit (6) is arranged so as to control the motor (5) and the linear ultrasound array (4) so that the motor moves the linear ultrasound array in a restricted rotation range, typically of 20 degrees, about the rotation axis (Y) during use.

**6.** The device as claimed in claim 4, wherein the control unit (6) is arranged so as to control the motor (5) and the linear ultrasound array (4) so that during use, after a first series of image acquisitions, the motor brings the linear ultrasound array into an initial position for a second series of image acquisitions, said initial position being determined at least on the basis of the images of the first series.

**7.** A method for guiding a medical probe for medical imaging (1) in order to bring the probe in proximity to an anatomical volume (101), the probe comprising means (4, 5) for acquiring images of the anatomical volume, and the method comprising the step of fixing to the probe at least one sensor (10) capable of generating signals representing at least one rotation of the probe in space, the method being **characterized in that** it comprises the steps of:

- processing the signals generated by the sensor in order to deduce therefrom at least one rotation of the probe in space,
- estimating a plurality of plausible positions of the probe relative to the anatomical volume on the basis of at least the deduction of the rotation of the probe in space,
- processing the images in order to localize the probe relative to the anatomical volume by determining the position of the probe relative to the anatomical volume on the basis of a reference image of the anatomical volume, of at least one image acquired by the image acquisition means, and of the plausible positions which have been determined.

**8.** The method as claimed in claim 7, wherein the step of estimating a plurality of plausible positions is carried out by means of a statistical or cinematic model (Mod(A,m)).

**9.** The method as claimed in claim 7, wherein the step of estimating a plurality of plausible positions is carried out on the basis of the deduction of only the rotation of the probe in space.

**10.** The method as claimed in claim 7, furthermore comprising the step of selecting only certain plausible positions of the probe relative to the anatomical volume in order to initialize the step of processing the images, in order to localize the probe relative to the anatomical volume by determining the position of the probe relative to the anatomical volume.

**11.** The method as claimed in claim 7, furthermore comprising the step of validating the position of the probe which has been determined relative to the anatomical volume.

**12.** The method as claimed in claim 7, comprising the successive steps of:

- acquiring at least one reference image of the anatomical volume ($I_r$) and determining at least one angular position of the probe in space, associated with the reference image, referred to as a reference angular position ($A_r$);
- acquiring at least a first image of the anatomical volume ($I_s$) and determining at least a first angular position ($A_s$) of the probe in space, associated with said image by processing the signals generated by the sensor in order to deduce therefrom the rotation of the probe in space:
- on the basis of the reference image ($I_r$), the reference angular position ($A_r$), the first image ($I_s$) and the first angular position ($A_s$), determining a finite number of positions ($T_m$) of the probe relative to the anatomical volume by means of a statistical or cinematic model (Mod(A,m)), so as to estimate a plurality of plausible positions of the probe relative to the anatomical volume;
- for each plausible position ($T_m$), calculating a similarity parameter ($S_m$) between the image of the anatomical volume ($I_s$) and a transformation of the reference image ($I_r$) on the basis of said plausible position;
- with the aid of the similarity parameters ($S_m$) calculated in the preceding step, selecting among the plausible positions ($T_m$) positions ($T_j$) closest to a real position of the probe relative to the anatomical volume;
- refining the determination of each position ($T_n$) of the probe relative to the anatomical volume by local optimization on each of the similarity parameters associated with the positions ($T_j$);
- among all the positions ($T_n$) thus refined, selecting the position ($T_k$) whose associated similarity parameter ($S_k$) is maximum after the local optimization step, said position being the position determined for the probe relative to the anatomical volume.

**13.** The method as claimed in claim 12, wherein the similarity parameter is calculated on the basis of a cross-correlation over the gray levels between the image of the anatomical volume ($I_s$) and the transformation of the reference image ($I_r$).

Fig.1

EP 2 854 668 B1

Fig.2

## Figure 3

Départ — 201

Acquisition I_r

Mesures A_I

Séquence d'initialisation

Calcul A_r — 202

Mémorisation I_r et A_r — 203

Séquence de déplacement

Départ — 301

Acquisition I_s

Mesures A_I

Calcul A_s — 302

Calcul A — 303

304

Détermination T_m
$T_m = Mod[A, m]$

Evaluation S_m
$S_m = Sim(I_s, I_r \ o \ T_m)$

305

Détermination Sj

Optimisation S_j
$<S_n, T_n = T_j \ o \ T_{max}>$
$= arg_{max_T}[T_j]sim(I_s, I_r \ o \ T_j \ o \ T)$

306 — Détermination S_k

Obtention T_k

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2920961
- US 6045508 A

- FR 2920961 A1